# EUROPEAN PATENT SPECIFICATION

(11) **EP 2 358 428 B1**
(45) Date of publication and mention of the grant of the patent: **12.12.2018**
(21) Application number: 09744262.8
(22) Date of filing: 15.10.2009
(51) Int. Cl.: A61N 1/05, A61N 1/36

(54) **IMPLANT ELECTRODE AND ACCESSORIES FOR USE IN ROBOTIC SURGERY**
IMPLANTATELEKTRODE UND ZUBEHÖR FÜR DEN EINSATZ IN ROBOTERGESTÜTZTER CHIRURGIE
ELECTRODE D'IMPLANT ET ACCESSOIRES DESTINÉS À ÊTRE UTILISÉS EN CHIRURGIE ROBOTIQUE

(30) Priority: 15.10.2008 US 105493 P
(43) Date of publication of application: 24.08.2011
(73) Proprietor: MED-EL Elektromedizinische Geräte GmbH, 6020 Innsbruck (AT)
(72) Inventor: JOLLY, Claude, 6020 Innsbruck (AT); SIEBER, Daniel, 6020 Innsbruck (AT)
(74) Representative: Downing, Michael Philip
(86) International application number: PCT/US2009/060787
(87) International publication number: WO 2010/053673

(56) References cited:
- EP-A2- 1 972 359
- US-A1- 2008 154 339

## Description

### FIELD OF THE INVENTION

The present invention relates to medical implants, and more specifically to an implant electrode and accessories for use in cochlear implant systems.

### BACKGROUND ART

Audio prosthesis electrodes such as cochlear implant electrodes are intended to be manually inserted into the inner ear cavity that will receive them. Usually cochlear implant electrodes are made of a somewhat soft silicone material which receives and protects the wires that connect to electrode contacts on the surface of the apical end of the electrode. There may also be a stylet to hold a pre-shaped electrode array section straight during insertion.

As used herein, the term "electrode array" refers to the apical end section of the implant electrode that penetrates into the cochlea scala of the inner ear. An electrode array has multiple electrode contacts on or slightly recessed below its outer surface for applying one or more electrical stimulation signals to target audio neural tissue. An "electrode lead" refers to the basal portion of the implant electrode that goes from the implant housing to the electrode array. It usually has no contact except perhaps a ground electrode and it encloses connecting wires delivering the electrical stimulation signals to the electrode contacts on the electrode array. The term "electrode" refers to the entire implant electrode from end to end, that is, the combination of the electrode array and the electrode lead.

US 2008/0154339 discloses an electrically nonconductive occludent constructed and arranged to forcefully infill a tissue opening so as to effectively prevent transport of electrical current, fluid and bacteria through the tissue opening. A cochlear implant comprises an elongate electrode carrier member having at least one electrode disposed thereon, wherein the carrier member is configured to traverse a cochleostomy to position the electrodes in the cochlea; and an electrically nonconductive occludent constructed and arranged to circumferentially surround a portion of the carrier member traversing the cochleostomy, and to forcefully infill cochleostomy thereby segregating perilymphatic canals of the cochlea from extracochlear regions.

### SUMMARY OF THE INVENTION

The invention is defined in the appended claims. Aspects, embodiments and examples disclosed of the present disclosure which do not fall under the scope of the appended claims do not form part of the invention and are merely provided for illustrative purposes.

The present invention is directed toward a guide for inserting an implantable electrode for a cochlear implant system, of the type including a basal electrode lead passing from an implant housing to a mastoid cortex surface for carrying one or more electrical stimulation signals from the implant housing. An apical electrode array fits through a cochleostomy opening into a cochlea scala and has multiple electrode contacts for applying the electrical stimulation signals to target neural tissue. A middle electrode section passes through the mastoid cortex and the middle ear to the cochleostomy opening for connecting the electrode lead and the electrode array.

The middle electrode section of the cochlear implant system may be straight or curved, and may be subdivided into a middle ear section and a mastoid section. The middle electrode element may include an outer tube support that provides structural stiffening, for example, based on at least one of a metallic, polymer, and textile material. Or the middle electrode element may include an inner core support such as a rod that provides structural stiffening, which may be based on at least one of a metallic, polymer, and textile material. The middle electrode section may have a larger diameter than the electrode lead and the electrode array. The middle electrode section may be rigid, and the electrode lead and the electrode array may be flexible. Or the electrode array may be rigid for direct insertion into the modiolus.

The electrode lead of the cochlear implant system may include one or more positioning knobs for moving the electrode into or out of the mastoid cortex surface. The electrode array may have a pre-shaped curve to fit into the cochlea, which may be activated by fluid heat, fluid hydration, or by release from a holding tube when inserted in the cochlea.

The present invention is directed to a guide for inserting a cochlear implant electrode insertion passage for insertion of a section of a stimulation electrode, said insertion passage extending from an outer mastoid cortex surface to a cochleostomy opening into a cochlea scala.

The guide member may include an interior volume through which the electrode array passes when inserted into the cochlea scala. The guide member may include a grooved channel that contains the section of the stimulation electrode. The guide member may form a stent, a tapered tube, a funnel shape, or be collapsible for insertion into the passage and then open like an umbrella. The guide member may be made of a mesh material. It may be rigid and/or removable. The guide member may be metallic, polymer or fabric, and may be straight or elbow shaped to turn the electrode array into the curve of the cochlea.

In some embodiments, the guide member may be externally lubricated to reduce friction when inserted or extracted, and/or internally lubricated to reduce friction when the electrode array slides through it. The guide member may have openings on its outer surface. It may include a stopper tip to prevent insertion of the guide member into the cochlea scala. There also may be positioning marks for use when inserting the guide member. There may be bifurcated sections to facilitate removal of the guide member from the passage after insertion of the electrode array in the cochlea scala. The guide member may include concentric coaxial tubes, which may have openings on their surfaces.

The guide member may be permanently implantable, for example, it may be biologically resorbable after insertion. The electrode array may be based on a thin film electrode. The guide member may include a detachable tip.

### BRIEF DESCRIPTION OF THE DRAWINGS

Figure 1 shows a cross-section of the skull bone structure near the cochlea.
Figure 2 shows an example of a passage through a portion of the skull bone to the cochlea according to one embodiment of the present invention.
Figure 3A-B shows introduction of an electrode array through the mastoid cortex into the cochlea scala according to an embodiment of the present invention.
Figure 4A shows a stimulation electrode according to one embodiment of the present invention.
Figure 4B shows use of an electrode guide for introduction of an electrode array through the mastoid cortex.
Figure 4C shows a implant electrode and electrode guide according to an embodiment.
Figure 4D shows another embodiment of a stimulation electrode according to the invention.
Figure 5A-B shows electrode guides according to various embodiments of the present invention.
Figure 6 shows a coaxial tube structure electrode guide.
Figure 7 shows an electrode guide having a stopper structure.
Figure 8 shows an elbow shaped electrode guide.
Figure 9 shows another elbow shaped electrode guide.
Figure 10 shows funnel shaped electrode guide.
Figure 11 shows a guide tip for an electrode guide.
Figure 12 shows various logical steps in one embodiment.

### DETAILED DESCRIPTION OF SPECIFIC EMBODIMENTS

Robotic surgery is being developed based on direct linear access from the skull surface to an inner ear opening. There is a need to adapt the implant electrode for either a non-manual insertion and/or an insertion through a robotically drilled narrow hole. Embodiments of the present invention are directed to an implant electrode that can be inserted into the cochlea through an electrode passage directly from the mastoid cortex through the middle ear and promontory bone into the cochlea scala, accessories for such inserting such an electrode, and the passage creation and electrode insertion processes.

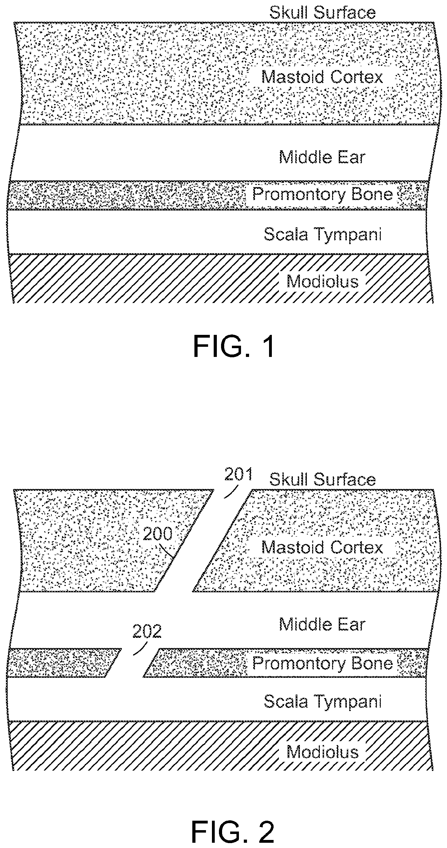

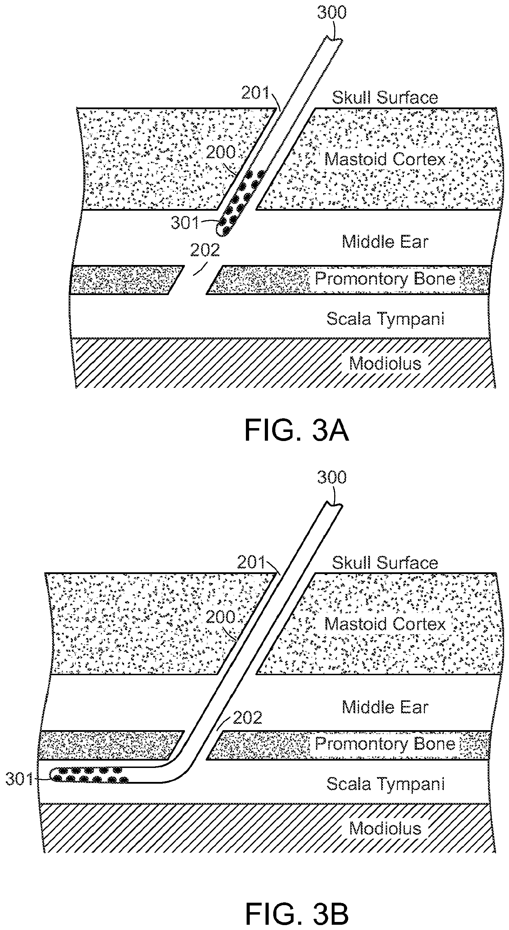

Figure 1 shows a cross-section of the skull bone structure near the cochlea with the skin removed. In order, the skull surface is the outer surface of the mastoid cortex bone, beneath which is the volume of the middle ear. The outer surface of the cochlea is at the promontory bone which encloses the interior scalae of the cochlea such as the scala tympani, which lies over the inner modiolus bone. During robotic surgery, to access the cochlea with a stimulation electrode, a narrow electrode passage 200 is drilled as shown in Figure 2 through the mastoid cortex and to directly access the promontory bone, where a cochleostomy can be remotely drilled to access the scala tympani. As shown in Figure 3 A-B, the implant electrode 300 with an electrode array 301 at its apical end, is inserted into the narrow opening 201 at the skull surface into the electrode passage 200, where it can be directed through the middle ear cavity, lined up with the cochleostomy 202 (or with the round window), and then pushed into the scala tympani without visual observation. Additional features can be incorporated into the implant electrode 300 to maneuver it past obstacles, for example, by rotating and/or pulling back.

In the example shown in Figs. 2 and 3, the electrode passage 200 is inclined at an acute angle to help the implant electrode 300 follow the cochlea scala as it is pushed in. If the electrode passage 201 is more perpendicular with respect to the scala, then the implant electrode 300 may collapse against the modiolus as it is inserted into the scala. Therefore, it is preferred that the angle of the electrode passage 200 be acute enough so that when the electrode array 301 is inserted into the scala it should follow the axial direction of the scala.

The ability to directly non-visually insert an implant electrode 300 though a pre-drilled electrode passage 200 into the cochlea scala allows major time savings during cochlear implant surgery and therefore potentially reduces the risk of operation complications and improves patient recovery. The implant electrode 300 can be inserted from relatively far away from the cochleostomy 202 by pushing it from the external aperture of the mastoid cortex opening 201. Forces are transmitted all the way to the distal tip of the electrode array 301 and insertion proceeds. An implant electrode 300 of this type is useful during robotic surgery that bypasses the prior technique of a mastoidectomy and posterior tympanotomy. The implant electrode 300 does not collapse in the middle ear if it is enclosed by a rigid detachable electrode guide. The electrode guide allows the tip of the electrode array 301 to easily line up with the cochleostomy 202. Another useful accessory is a stent-like mesh that is deployed to allow the implant electrode 300 to take the correct direction and orientation toward the longitudinal axis of the scala.

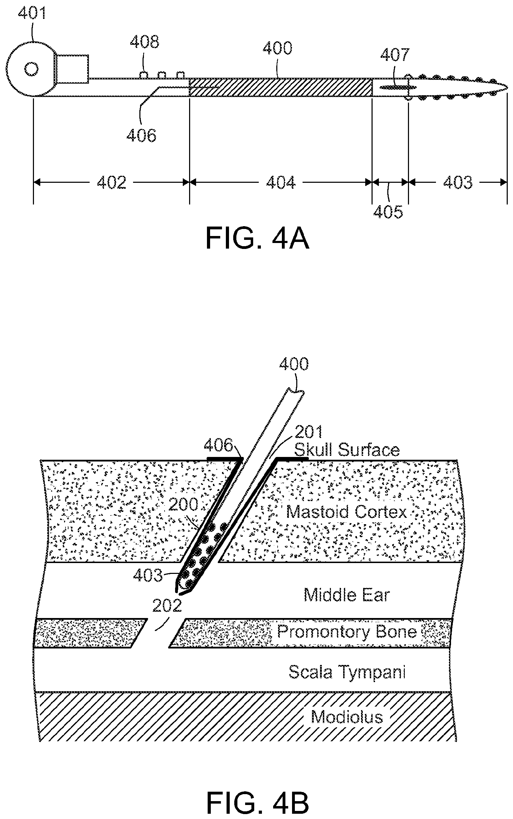

Figure 4A shows one example of a complete implant electrode 400 according to one specific embodiment, which is attached to an implant housing 401 which processes the electrical stimulation signals to be applied by the implant electrode 400 to target audio neural tissue. A basal electrode lead 402 section passes from the implant housing 401 to the surface opening of the mastoid cortex surface. An apical electrode array 403 section fits through the cochleostomy opening in the promontory bone into a cochlea scala and has multiple electrode contacts for applying the electrical stimulation signals to target neural tissue. In specific embodiments, the electrode array 403 and its electrode contacts may be similar to what has been used before. In some embodiments, the tip of the electrode array 403 may have a pre-shaped curve to accommodate a sharp turn when it enters the scala through the cochleostomy. The pre-shaped curve may be activated in various ways such as by heat or hydration from the scala fluid, or by simple release from the electrode guide.

A middle electrode section is subdivided into a mastoid section 404 which passes through the mastoid cortex, and a middle ear section 405 which passes through the middle ear to the cochleostomy opening for connecting the electrode lead 402 and the electrode array 403. In the embodiment shown in Fig. 4A, the mastoid section 404 and the middle ear section 405 are stiff and rigid to allow the electrode array 403 to be pushed through the electrode passage. The stiffness allows the implant electrode 400 to be easily rotated because rotational movement is transmitted from the surface of the skull section several centimeters down to the apical end of the electrode array 403 thereby allowing maneuverability so as to overcome any obstacle it may encounter such as a curvature, a blockage, a basilar membrane, or a modiolus curvature where the electrode array 403 could get stuck. Depending on the specific circumstances, the electrode array 403 may be normally flexible for insertion into the cochlea scala, or it may also be stiff for use as a penetrating electrode arrangement to stimulate target neural tissue within the modiolus. Penetrating electrodes are described more fully in U.S. Provisional Patent Application 61/097,343, filed September 16, 2008, which is hereby incorporated by reference. The electrode lead 402 needs normal flexibility to allow good placement of the implant housing 401 on the skull bone to wind back any excess electrode lead; for example, into a hole specially drilled on the skull for this purpose. The embodiment shown in Fig. 4A also includes positioning knobs 408 for pushing the implant electrode 400 into or pulling it out of the opening in the mastoid cortex.

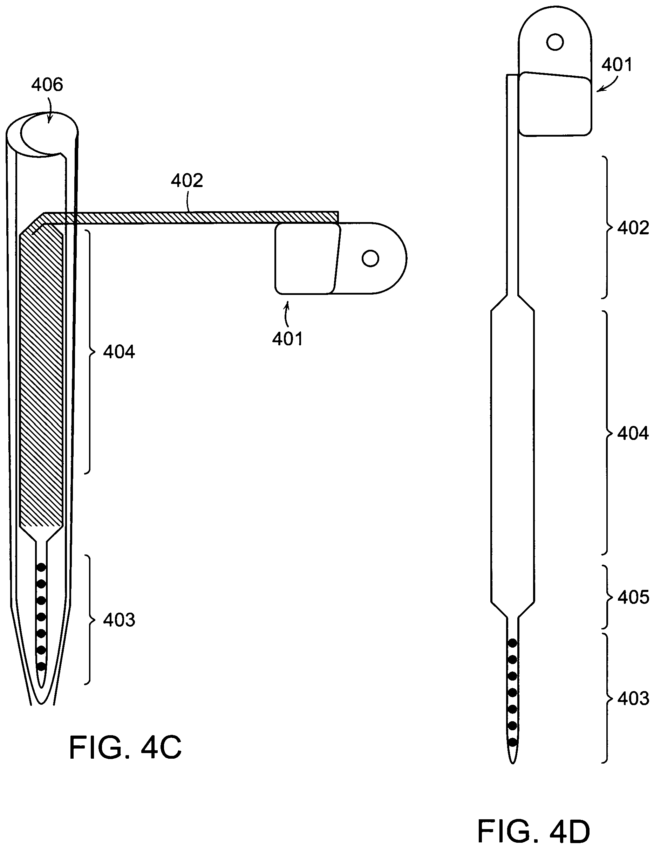

There are several specific ways to achieve the desired degree of stiffness in the middle electrode section. Either or both of the mastoid section 404 and the middle ear section 405 can be fabricated from a stiffer polymer material than the highly flexible silicone typically used for implant electrodes-for example, a stiff polymer may be used, such as metallic, polymer or textile tubing, a stiffener core, a rod embedded in silicone, and/or anchors or some combination thereof. Figure 4D shows that the stiff middle electrode section of the mastoid section 404 and the middle ear section 405 may have a larger diameter to be more rigid than the smaller diameter and more flexible electrode array 403 and electrode lead 402. In addition or alternatively, a permanent stiffening feature 407 such as an internal stiffening rod may be incorporated into one or more sections of the implant electrode 400.

There may also be one or more removable stiffening features such as an electrode guide 406 shown in Fig. 4A and 4B made of metal, polymer, or fabric. The rigid electrode guide 406 in the form of a tapered open channel defines and provides rigid continuity for the electrode passage 200 from the narrow opening 201 in the skull surface, through the mastoid cortex and middle ear to the cochleostomy opening 202. This provides stability so that the electrode array 403 can be safely and easily inserted into the desired location in the cochlea scala. In specific embodiments, the outer surface of the electrode guide 406 may be externally lubricated to reduce friction when inserted or extracted. The electrode guide 406 may also be internally lubricated to reduce friction when the electrode 400 slides through it.

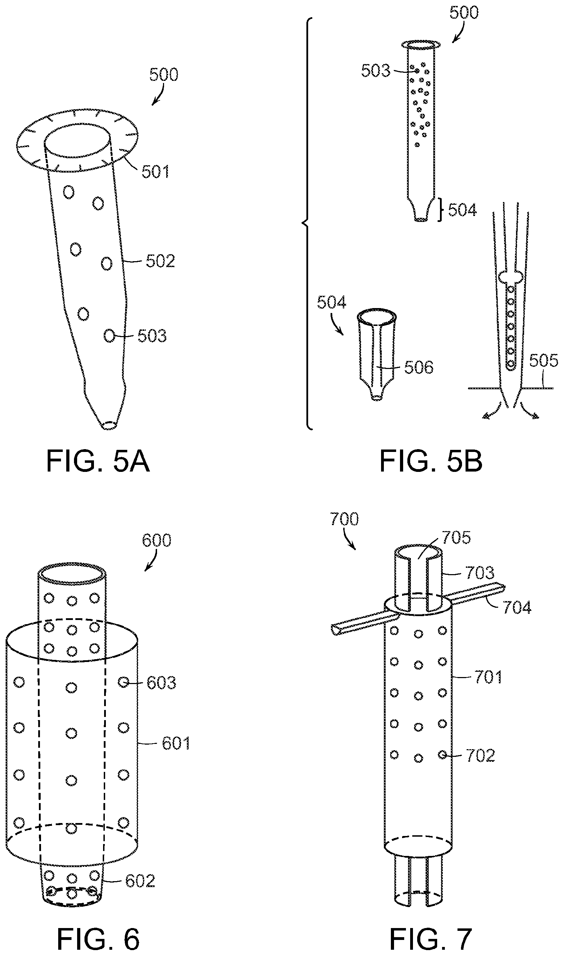

Enclosed within the electrode guide 406 are the middle electrode section 404 and 405 and the electrode array 403, the latter of which is inserted through the cochleostomy opening in the promontory bone into the cochlea scala. The channel opening in the electrode guide 406 does not allow the rigid and larger diameter middle electrode section to come out, but does allow the smaller diameter basal portion of the electrode lead on the skull to easily come out, making it easy to remove the electrode guide 406 once the electrode array has been inserted into the cochlea scala. The electrode guide 406 is removed simply by pulling it back out of the mastoid opening while holding the thinner part of the electrode lead at some angle as shown on Figure 4C. The electrode guide 406 should be fairly rigid, tapered, and adapted to be pulled back through the opening 201 in the mastoid cortex and disconnected from the electrode lead 400. Removal of the electrode guide may be aided by use of an array slot 506 as shown in the detail at the bottom of Figure 5B. Alternatively, the electrode guide 406 may be designed and manufactured from a bio-resorbable material.

Figure 5A shows an embodiment of simple rigid tube-shaped electrode guide 500 having an elongated tubular section 502 that encloses the middle section of the electrode and the electrode array. A flange opening 501 fits over the opening in the mastoid cortex. Hole openings 503 in the outer surface of the tubular guide member 502 facilitate regrowth of the surrounding tissue and anchors the tubular guide member 502 in a fixed position, as well as allowing extra-cellular fluid to circulate and irrigate the interior volume to avoid developing infections. In some embodiments, there may usefully be a stopper tip 505 structure as shown in Fig. 5B which prevents insertion of the guide member beyond the cochleostomy opening and into the cochlea scala. In the event that the implant electrode needs to be removed, the electrode guide 500 can be left permanently implanted in the patient and a new electrode inserted through it. Additionally the electrode guide 500 can be marked with a scalar markings to facilitate use of information from pre-operative imaging during insertion.

Figure 6 shows an electrode guide 600 having a coaxial double tube arrangement where an inner tube 602 and an outer tube 601 are fitted together. Both or either of the inner tube 602 and outer tube 601 may be perforated by a series of hole openings 603, which allow tissue regrowth to occur and thereby anchor the electrode guide 600 in a fixed position, as well as allow extra-cellular fluid to circulate and irrigate the inner tube 602 and thereby preventing infections from developing. All or part of the electrode guide 600 may be in the form of a mesh and deployed as a stent. Instead of hole openings 603, some embodiments may have or open slots or may be mesh-like.

Figure 7 shows a variation of a double tube electrode guide 700 having a stopper bar 704 near the opening in the mastoid cortex. A slot 705 on the inner tube 703 allows removal of the guide from the implant electrode as it is being pulled back.

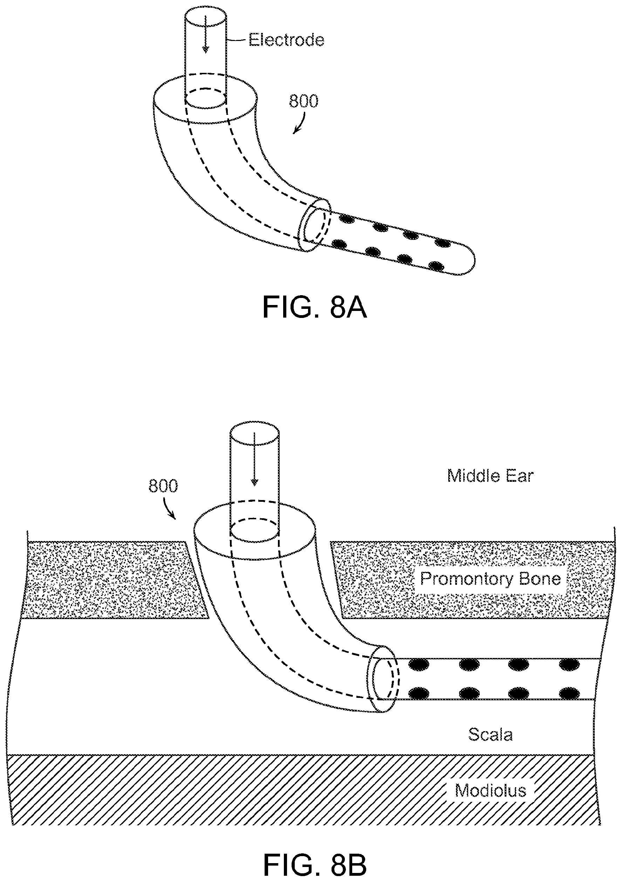

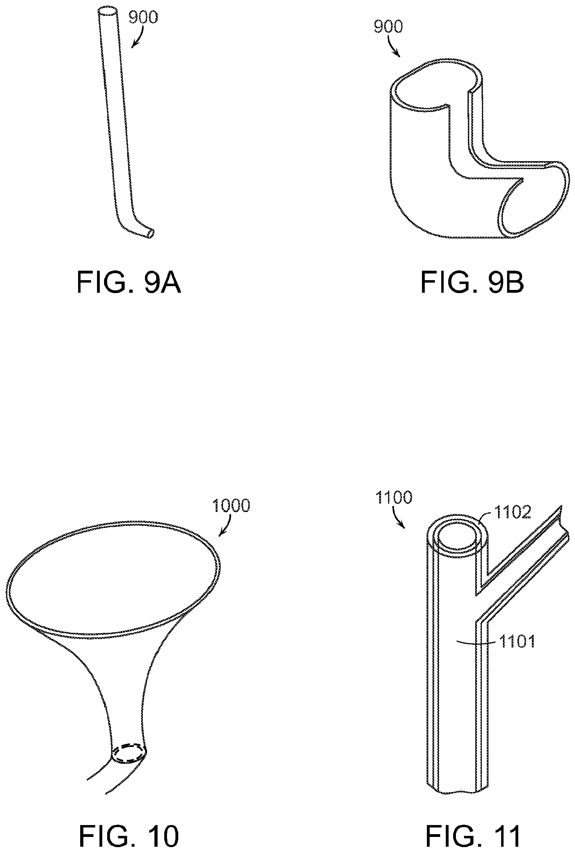

Embodiments of the present invention also include an electrode guide accessory for placement in the cochleostomy opening for guiding the electrode array into the cochlea scala. Such an electrode guide is shown in Figure 8 where a stent-type mesh cochleostomy guide 800 has an elbow-shaped curve to direct the electrode array towards the long axis of the cochlea scala as desired. Figure 8B shows a variation of a cochleostomy guide 800 in the same elbow-shape with a smooth outer surface. Because of the elbow shape, pushing down and in on the outer portion of an implant electrode is translated in direction so that the distal end of the electrode array enters the cochlea scala as shown along its directional axis. In some embodiments, the cochleostomy guide 800 may be deployable from within an electrode guide that is inserted into the electrode passage-e.g., like a collapsed umbrella shape that opens when in position. The cochleostomy guide 800 may be made of polymer, metal (e.g., nitinol), or fabric. In specific embodiments, the cochleostomy guide 800 may be removable or may be permanently implantable, e.g., by being bio-resorbable. Figure 9A shows an alternative cochleostomy guide 900 in the form of a distal tip section for an entire electrode guide as described above, which may or may not be detachable. Figure 9B shows an alternative embodiment of a cochleostomy guide 900 having a slot on top for easy removal after insertion of the electrode array.

Figure 10 shows an embodiment a funnel-shaped cochleostomy guide 1000 that can be deployed in the middle ear to facilitate aligning the implant electrode with the cochleostomy opening. The funnel-shaped cochleostomy guide 1000 may be a mesh-type structure, e.g., a super-elastic nitinol, and during insertion in the mastoid hole, it is collapsible opens in the middle ear like an umbrella once it is inserted. The funnel-shaped cochleostomy guide 1000 may be in the form of a distal tip section for an entire electrode guide as described above, which may or may not be detachable.

Figure 11 shows yet another arrangement of an electrode guide 1100 where the implant electrode 1101 is bifurcated near the skull as shown. The bifurcated form allows a slot or notch 1102 in the electrode guide 1100 to be used to remove it from the implant electrode 1101 with minimum disturbance.

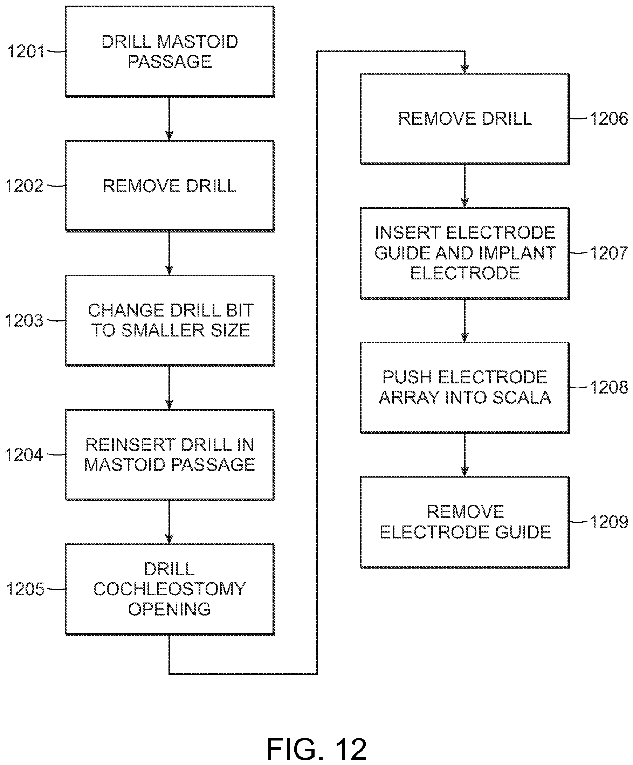

Embodiments of the present invention also include methods to insert an implant electrode (or drug delivery catheter) directly through an electrode passage in the skull. In some embodiments, this may be performed robotically and may not even require visual observation of the process. Figure 12 shows various logical steps in one embodiment of such a method where initially, a passage is drilled through the mastoid cortex to the middle ear, step 1201. This mastoid passage should be slightly larger in diameter than the combined diameter of the electrode guide and implant electrode. The site and direction of the mastoid passage is determined based on pre-operative scans and markers, using standard techniques. Then the drill is removed, step 1202, and the drill bit changed to a smaller size which is slightly larger in diameter than the electrode array, step 1203. The drill is reinserted in the mastoid cortex opening, step 1204, possibly using a drill guide such as a catheter tube that fits in the mastoid passage. The cochleostomy opening in the promontory bone is then drilled, step 1205. The drill is removed, step 1206, and the electrode guide containing the implant electrode is inserted into the mastoid opening, step 1207 and pushed into the mastoid passage until resistance is met. At that point, the electrode guide is held in place while the implant electrode is blindly pushed into the scala, step 1208. Once the electrode array is fully inserted, the electrode guide is pulled back or removed, step 1209.

Typically, the opening in the mastoid cortex and the mastoid passage may be larger than the cochleostomy opening. It may be helpful to have a feature to accommodate this change in size to allow correct alignment of the axis of the electrode array with the center of the cochleostomy when inserted. This may also be needed when using the smaller diameter drill bit to form the cochleostomy opening-the drill bit needs to be centered in the mastoid passage.

In an alternative embodiment, the diameter of the mastoid cortex may be kept as small as possible to avoid sensitive structures (such as the facial nerve tissue and chordu tympani nerve tissue) when drilling through to the middle ear and then the cochleostomy. A thin film electrode can then be inserted in the electrode guide and into the cochlea scala. In another example, another step may be added to deploy a stent-like mesh curved guide in the cochleostomy, to better direct the electrode along the long axis of the scala.

It is understood that lubricants, lubricious coating, anti inflammatory coating, may be used in combination with the device and accessories described here. It is also understood that the implant electrode, drug delivery catheter, and the various accessories may be beneficial if using some type of endoral surgical approach, canal wall drill out, etc.

Although various exemplary embodiments of the invention have been disclosed, it should be apparent to those skilled in the art that various changes and modifications can be made which will achieve some of the advantages of the invention without departing from the true scope of the invention.

## Claims

1. An electrode guide for inserting a cochlear implant electrode array into a cochlea scala, the guide comprising:
an elongated guide member (406, 500, 600, 700, 800, 900, 1000, 1100) configured to define an insertion passage for an electrode array, said insertion passage extending through an outer mastoid cortex surface to a cochleostomy opening into a cochlea scala.

2. An electrode guide according to claim 1, wherein the guide member includes an interior volume through which the electrode array passes when inserted into the cochlea scala.

3. An electrode guide according to claim 1, wherein the guide member includes a grooved channel through which the electrode array passes when inserted into the cochlea scala.

4. An electrode guide according to claim 1, wherein the guide member forms a tapered tube.

5. An electrode guide according to claim 1, wherein the guide member forms a funnel shape.

6. An electrode guide according to claim 5, wherein the guide member is collapsible for insertion into the passage and then opens like an umbrella

7. An electrode guide according to claim 1, wherein the guide member is made of a mesh material.

8. An electrode guide according to claim 1, wherein the guide member forms a stent.

9. An electrode guide according to claim 1, wherein the guide member is removable.

10. An electrode guide according to claim 1, wherein the guide member is rigid.

11. An electrode guide according to claim 1, wherein the guide member is elbow shaped to turn the electrode array into the curve of the cochlea.

12. An electrode guide according to claim 1, wherein the guide member is internally lubricated to reduce friction when the electrode array slides through it.

13. An electrode guide according to claim 1, wherein the guide member includes openings on its outer surface.

14. An electrode guide according to claim 1, wherein the guide member includes a stopper tip to prevent insertion of the guide member into the cochlea scala.

15. An electrode guide according to claim 1, wherein the guide member includes positioning marks for use when inserting the guide member.

16. An electrode guide according to claim 1, wherein the guide member has bifurcated sections to facilitate removal of the guide member from the passage after insertion of the electrode array in the cochlea scala.

17. An electrode guide according to claim 1, wherein the guide member includes a plurality of concentric coaxial tubes.

18. An electrode guide according to claim 17, wherein the tubes have a plurality of openings on their surfaces.

19. An electrode guide according to claim 1, wherein the guide member is configured to be permanently implantable.

20. An electrode guide according to claim 1, wherein the guide member is adapted to be biologically resorbable after insertion.

21. An electrode guide according to claim 1, wherein the guide member includes a detachable tip.

22. An electrode guide according to claim 1, in combination with an electrode array.

23. The combination of an electrode guide and an electrode array according to claim 22, wherein the electrode array comprises a thin film electrode.

## Patentansprüche

1. Elektrodenführung zum Einführen einer kochlearen Implantatelektrodenanordnung in eine Kochlea-Scala, die Führung umfassend:
ein längliches Führungselement (406, 500, 600, 700, 800, 900, 1000, 1100), das ausgelegt ist, einen Einführungsdurchgang für eine Elektrodenanordnung zu definieren, wobei der Einführungsdurchgang durch eine äußere Mastoid-Cortex-Fläche zu einer Kochleostomieöffnung in eine Kochlea-Scala verläuft.

2. Elektrodenführung nach Anspruch 1, wobei das Führungselement ein Innenvolumen enthält, durch welches die Elektrodenanordnung hindurchgeht, wenn sie in die Kochlea-Scala eingeführt wird.

3. Elektrodenführung nach Anspruch 1, wobei das Führungselement einen geriffelten Kanal enthält, durch welchen die Elektrodenanordnung hindurchgeht, wenn sie in die Kochlea-Scala eingeführt wird.

4. Elektrodenführung nach Anspruch 1, wobei das Führungselement eine verjüngte Röhre bildet.

5. Elektrodenführung nach Anspruch 1, wobei das Führungselement eine Trichterform bildet.

6. Elektrodenführung nach Anspruch 5, wobei das Führungselement zur Einführung in den Durchgang zusammenklappbar ist und sich dann wie ein Regenschirm öffnet.

7. Elektrodenführung nach Anspruch 1, wobei das Führungselement aus einem Maschenmaterial hergestellt ist.

8. Elektrodenführung nach Anspruch 1, wobei das Führungselement einen Stent bildet.

9. Elektrodenführung nach Anspruch 1, wobei das Führungselement entfernbar ist.

10. Elektrodenführung nach Anspruch 1, wobei das Führungselement hart ist.

11. Elektrodenführung nach Anspruch 1, wobei das Führungselement ellenbogenförmig ist, um die Elektrodenanordnung in die Krümmung der Kochlea zu drehen.

12. Elektrodenführung nach Anspruch 1, wobei das Führungselement intern geschmiert ist, um Reibung zu verringern, wenn die Elektrodenanordnung durch es hindurchgleitet.

13. Elektrodenführung nach Anspruch 1, wobei das Führungselement Öffnungen auf seiner Außenfläche enthält.

14. Elektrodenführung nach Anspruch 1, wobei das Führungselement eine Stopperspitze enthält, um die Einführung des Führungselements in die Kochlea-Scala zu verhindern.

15. Elektrodenführung nach Anspruch 1, wobei das Führungselement Positionierungsmarkierungen zur Nutzung enthält, wenn das Führungselement eingeführt wird.

16. Elektrodenführung nach Anspruch 1, wobei das Führungselement gegabelte Abschnitte aufweist, um das Entfernen des Führungselements aus dem Durchgang nach der Einführung der Elektrodenanordnung in die Kochlea-Scala zu erleichtern.

17. Elektrodenführung nach Anspruch 1, wobei das Führungselement mehrere konzentrische koaxiale Röhren enthält.

18. Elektrodenführung nach Anspruch 17, wobei die Röhren mehrere Öffnungen auf ihren Oberflächen aufweisen.

19. Elektrodenführung nach Anspruch 1, wobei das Führungselement ausgelegt ist, dauerhaft implantierbar zu sein.

20. Elektrodenführung nach Anspruch 1, wobei das Führungselement angepasst ist, nach der Einführung biologisch resorbierbar zu sein.

21. Elektrodenführung nach Anspruch 1, wobei das Führungselement eine abnehmbare Spitze enthält.

22. Elektrodenführung nach Anspruch 1 in Kombination mit einer Elektrodenanordnung.

23. Kombination einer Elektrodenführung und einer Elektrodenanordnung nach Anspruch 22, wobei die Elektrodenanordnung eine Dünnschichtelektrode umfasst.

## Revendications

1. Guide d'électrodes pour insérer un faisceau d'électrodes comme implant cochléaire dans une rampe de cochlée, le guide comprenant un élément allongé (406, 500, 600, 700, 800, 900, 1000, 1100) de guide configuré pour délimiter un passage d'insertion pour un faisceau d'électrodes, ledit passage d'insertion traversant une surface extérieure du cortex mastoïde jusqu'à une ouverture de cochléostomie pénétrant dans une rampe de cochlée.

2. Guide d'électrodes selon la revendication 1, dans lequel l'élément de guide comprend un volume intérieur par lequel passe le faisceau d'électrodes quand on l'insère dans la rampe de cochlée.

3. Guide d'électrodes selon la revendication 1, dans lequel l'élément de guide comprend un canal rainuré par lequel passe le faisceau d'électrodes quand on l'insère dans la rampe de cochlée.

4. Guide d'électrodes selon la revendication 1, dans lequel l'élément de guide forme un tube conique.

5. Guide d'électrodes selon la revendication 1, dans lequel l'élément de guide présente une forme d'entonnoir.

6. Guide d'électrodes selon la revendication 5, dans lequel l'élément de guide est rabattable pour l'insertion dans le passage et puis s'ouvre comme un parapluie.

7. Guide d'électrodes selon la revendication 1, dans lequel l'élément de guide est fait d'une matière à mailles.

8. Guide d'électrodes selon la revendication 1, dans lequel l'élément de guide constitue un stent.

9. Guide d'électrodes selon la revendication 1, dans lequel l'élément de guide est amovible.

10. Guide d'électrodes selon la revendication 1, dans lequel l'élément de guide est rigide.

11. Guide d'électrodes selon la revendication 1, dans lequel l'élément de guide a une forme de coude pour faire pénétrer le faisceau d'électrodes dans la courbe de la cochlée.

12. Guide d'électrodes selon la revendication 1, dans lequel l'élément de guide est lubrifié à l'intérieur pour réduire le frottement quand le faisceau d'électrodes coulisse à travers lui.

13. Guide d'électrodes selon la revendication 1, dans lequel l'élément de guide comprend des ouvertures sur sa surface extérieure.

14. Guide d'électrodes selon la revendication 1, dans lequel l'élément de guide comprend un embout butoir pour empêcher l'insertion de l'élément de guide dans la rampe de cochlée.

15. Guide d'électrodes selon la revendication 1, dans lequel l'élément de guide comprend des marques de mise en place à utiliser quand on insère l'élément de guide.

16. Guide d'électrodes selon la revendication 1, dans lequel l'élément de guide comporte des sections bifurquées pour faciliter le retrait de l'élément de guide après insertion du faisceau d'électrodes dans la rampe de cochlée.

17. Guide d'électrodes selon la revendication 1, dans lequel l'élément de guide comprend une pluralité de tubes coaxiaux concentriques.

18. Guide d'électrodes selon la revendication 17, dans lequel les tubes ont une pluralité d'ouvertures sur leur surface.

19. Guide d'électrodes selon la revendication 1, dans lequel l'élément de guide est configuré pour pouvoir être implanté de façon permanente.

20. Guide d'électrodes selon la revendication 1, dans lequel l'élément de guide est apte à pouvoir être résorbée biologiquement après insertion.

21. Guide d'électrodes selon la revendication 1, dans lequel l'élément de guide comprend un embout amovible.

22. Guide d'électrodes selon la revendication 1, en combinaison avec un faisceau d'électrodes.

23. Combinaison d'un guide d'électrodes et d'un faisceau d'électrodes selon la revendication 22, dans laquelle le faisceau d'électrodes comprend une électrode en couches minces.
